# EUROPÄISCHE PATENTSCHRIFT

(11) **EP 2 533 724 B1**
(45) Veröffentlichungstag und Bekanntmachung des Hinweises auf die Patenterteilung: **11.05.2016**
(21) Anmeldenummer: 11822804.8
(22) Anmeldetag: 17.11.2011
(51) Int. Cl.: A61F 2/16, A61F 2/14

(54) **VORRICHTUNG ZUM BEREITSTELLEN UND ZUM EINBRINGEN EINES TRANSPLANTATS ODER EINES IMPLANTATS IN DEN LEBENDEN KÖRPER, INSBESONDERE FÜR OPHTHALMOLOGISCHE EINGRIFFE**
DEVICE FOR PREPARING AND INTRODUCING A TRANSPLANT OR AN IMPLANT INTO A LIVING BODY, IN PARTICULAR FOR OPHTHALMOLOGICAL INTERVENTIONS
DISPOSITIF POUR PRÉPARER ET INTRODUIRE UN GREFFON OU UN IMPLANT DANS UN CORPS VIVANT, EN PARTICULIER POUR DES INTERVENTIONS OPHTALMOLOGIQUES

(30) Priorität: 17.11.2010 DE 102010051458
(43) Veröffentlichungstag der Anmeldung: 19.12.2012
(73) Patentinhaber: Geuder AG, 69126 Heidelberg (DE)
(72) Erfinder: SZURMAN, Peter, 66121 Saarbrücken (DE)
(74) Vertreter: Patent- und Rechtsanwälte Ullrich & Naumann
(86) Internationale Anmeldenummer: PCT/DE2011/050048
(87) Internationale Veröffentlichungsnummer: WO 2012/065602

(56) Entgegenhaltungen:
- US-A1- 2009 270 876
- US-B1- 6 605 093

## Beschreibung

Die vorliegende Erfindung betrifft eine Vorrichtung zum Bereitstellen und zum Einbringen eines Transplantats oder eines Implantats in den lebenden Körper, in Kombination einer Spritze zum Betätigen der Vorrichtung, insbesondere für ophthalmologische Eingriffe, und insbesondere zur Durchführung einer DMEK-Operation.

Die Transplantation der menschlichen Hornhaut gehört zu den am häufigsten und erfolgreichsten durchgeführten transplantationschirurgischen Eingriffen. Die Transplantation der Hornhaut unter Verwendung aller ihrer Schichten (Epithel, Bowman-Membran, Stroma, Descemet-Membran und Endothelzellschicht) im Rahmen einer perforierenden Keratoplastik ist seit über 100 Jahren bekannt. Mit dieser Operationsmethode werden im Allgemeinen gute Ergebnisse erzielt, jedoch ist die Rekonvaleszenz sehr langwierig und eine Visuserholung kann üblicherweise endgültig erst nach Entfernung des zweiten Fadens erreicht werden. Dies kann eine Zeitspanne von bis zu 18 Monaten nach dem Eingriff ausmachen.

Gut die Hälfte aller Hornhauttransplantationen wird wegen Erkrankungen des Hornhautendothels durchgeführt. Für diese Erkrankungen würde sich prinzipiell ein schichtspezifischer Ersatz der Hornhaut eignen. Geeignete Techniken hierfür liegen mittlerweile vor. Durch die Transplantation der Descemet'schen Membran mit einer anhängenden Stromalamelle (DSAEK) kann seit einigen Jahren eine im Vergleich mit der perforierenden Keratoplastik viel schnellere Visuserholung und demzufolge eine bessere Patientenzufriedenheit erreicht werden.

Zur Therapie bei Erkrankungen der Hornhaut, welche das Hornhautendothel betreffen, ist des Weiteren eine neuartige, spezielle Form der Hornhauttransplantation beschrieben worden. Dabei handelt es sich um die Descemet Membrane Endothelial Keratoplasty (DMEK). Im Zuge eines solchen Eingriffs können die erkrankten Endothel-Zellen einschließlich der darunter liegenden Descemet-Membran entfernt und durch eine Descemet-Membran mit gesundem Hornhautendothel eines Spenders ersetzt werden. Auf eine perforierende Keratoplastik kann dabei verzichtet werden, vielmehr lässt sich das Transplantat durch eine vergleichsweise kleine Inzision in die vordere Augenkammer transferieren und durch sehr vorsichtige Manipulation ausbreiten. Danach wird das Membran-Präparat mittels Luftzugabe am hinteren Stroma fixiert.

Eine erfolgreiche DMEK-Operation ermöglicht dem Patienten eine sehr schnelle visuelle Rehabilitation. Es ist davon auszugehen, dass der Patient schon nach ca. zwei Monaten nach dem Eingriff eine volle Sehschärfe erreicht.

Allgemein sind eine ganze Reihe chirurgischer und insbesondere ophthalmologischer Eingriffe bekannt oder denkbar, bei denen ein Transplantat oder ein Implantat bereitgestellt und in den lebenden Körper eingebracht wird. Dabei ist es im Sinne einer raschen Rekonvaleszenz des Patienten - wie oben anhand der DMEK-Operation beispielhaft ausgeführt - vorteilhaft, ein Transplantat oder Implantat durch eine Inzision begrenzter, und insbesondere möglichst kleiner Größe in den lebenden Körper einzubringen.

Wird eine geeignete Vorrichtung zum Einbringen eines Transplantats oder eines Implantats verwendet, stellt sich jedoch das Problem, das Transplantat oder Implantat ohne jede Beschädigung und ggf. in einer konkreten Lage bzw. Position in dieser Vorrichtung bereitzustellen. Insbesondere für den Fall, dass die Vorrichtung zum Einbringen eines Transplantats in eine sehr kleine Inzision im Körper des Patienten geeignet ist, kann das Transplantat aufgrund der zwangsläufig geringen Ausmaße einer solchen Vorrichtung bereits beim Bereitstellen in der Vorrichtung beschädigt oder zerstört werden. Ggf. ist ein Bereitstellen des Transplantats in der Vorrichtung aufgrund der geringen Abmessungen der Vorrichtung bereits anfangs unmöglich.

Aus US 6,605,093 B1 ist eine Vorrichtung zum Bereitstellen und zum Einbringen eines Transplantats oder eines Implantats für ophthalmologische Eingriffe bekannt, die mit einer spritzenartigen Einheit zusammenwirkt. Die eigentliche Vorrichtung und die Spritzeneinheit werden miteinander verschraubt. Die Vorrichtung nebst Spritzeneinheit sind aufwändig in der Konstruktion und komplex in der Handhabung, zumal eine schraubende Betätigung/Handhabung erforderlich ist.

Aus US 2009/0270876 A1 ist eine ähnlich komplexe Vorrichtung bekannt, bei der zur Betätigung darauf abgestimmte Betätigungsmittel erforderlich sind. Eine kodierte Aufnahme erfordert exakt angepasste Bestätigungsmittel, die in der Konstruktion aufwändig sind. Da die Vorrichtung stets "von hinten" beladen wird, ist die Handhabung abermals aufwändig.

Der vorliegenden Erfindung liegt nun die Aufgabe zugrunde, eine Vorrichtung anzugeben, welche sich sowohl für das schonende Bereitstellen als auch das exakte Einbringen eines Transplantats oder eines Implantats in den lebenden Körper bei einfachster Konstruktion und sicherer Handhabung eignet.

Diese Aufgabe ist durch eine Vorrichtung mit den Merkmalen des Anspruchs 1 gelöst. Danach ist die Vorrichtung mit einer Spritze kombiniert, wobei die Vorrichtung eine Kartusche oder eine Hülse mit zwei gegenüberliegenden, endseitigen Öffnungen aufweist, wobei die erste Öffnung einen größeren Durchmesser aufweist als die zweite Öffnung, wobei der äußere Rand der zweiten Öffnung in die Düse der Spritze einsteckbar ist und die erste Öffnung auf die Düse der Spritze aufsteckbar ist, so dass das Transplantat oder Implantat mitsamt Flüssigkeit durch die erste Öffnung in die Kartusche eingezogen wird, indem der Kolben der an die zweite Öffnung angeschlossenen Spritze nach hinten gezogen wird, und das Transplantat oder Implantat mitsamt der im Inneren der Vorrichtung vorhandenen Flüssigkeit aus der Kartusche ausstoßbar ist, indem die erste Öffnung auf die Düse der Spritze aufgesteckt und durch Betätigen des Kolbens der Spritze Flüssigkeit in die Kartusche gepresst wird, wodurch die im Inneren der Vorrichtung vorhandene Flüssigkeit zusammen mit dem Transplantat oder Implantat durch eine geeignete Inzision in den Körper eines Patienten befördert wird. Des Weiteren soll ein Set, umfassend die Vorrichtung und die Spritze, angegeben werden.

Vorteilhafte Ausgestaltungen und Weiterbildungen der erfindungsgemäßen Vorrichtung sind den hierzu nachgeordneten Ansprüchen entnehmbar. Mit dem nebengeordneten Anspruch 9 wird ein gebrauchsfertiges Set vorgeschlagen, welches eine erfindungsgemäße Vorrichtung nebst Spritze umfasst. Eine zweckmäßige Ausführungsform des gebrauchsfertigen Sets ist Gegenstand des hierzu nachgeordneten Anspruchs.

Erfindungsgemäß ist zunächst eine Vorrichtung vorgesehen, mit bzw. in der sowohl eine Aufnahme des Transplantats bzw. Implantats und damit eine Bereitstellung als auch ein Einbringen in den Körper des Patienten erfolgen kann.

Im Folgenden wird dabei hauptsächlich von dem Bereitstellen und Einbringen eines Transplantats gesprochen. Dabei wird jedoch stets vorausgesetzt, dass anstelle eines Transplantats in für den Fachmann offensichtlicher Weise auch ein geeignetes Implantat bereitgestellt bzw. eingebracht werden kann. Dies wird jedoch nicht anlässlich jedes Einzelfalles ausdrücklich wiederholt.

Das Transplantat oder Implantat lässt sich durch die erste, größere Öffnung in die Vorrichtung einbringen, ohne dass die Gefahr einer Beschädigung - insbesondere durch Wand- oder Randkontakt mit der Vorrichtung - erfolgt. Dabei wird das Transplantat zusammen mit umgebender Flüssigkeit - bspw. Kochsalzlösung - in die erste Öffnung der Vorrichtung eingesogen.

Durch die erste, größere Öffnung der Vorrichtung lassen sich auch Transplantate einbringen und bereitstellen, welche im ursprünglichen Zustand größere Abmessungen aufweisen als der Durchmesser der Inzision, durch welche ein Einbringen des Transplantats in den Körper des Patienten geplant ist.

In weiter erfindungsgemäßer Weise bietet die Vorrichtung die Möglichkeit, das innerhalb der Vorrichtung bereitgestellte Transplantat nunmehr durch die zweite, kleinere Öffnung der Vorrichtung in den Körper des Patienten einzubringen, und zwar durch eine möglichst kleine Inzision. Der der zweiten Öffnung zugeordnete Abschnitt der Vorrichtung kann demnach als kanülenartiges Ende oder an eine geeignete Kanüle anschließbar ausgestaltet sein. Im Allgemeinen ist der Abschnitt der Vorrichtung, welcher die zweite, kleinere Öffnung aufweist, so ausgestaltet, dass ein Einbringen des Transplantats oder Implantats in den Körper des Patienten über die zweite Öffnung ermöglicht wird.

Erfindungsgemäß ist in besonders raffinierter Weise ermöglicht, dass ein geeignetes Transplantat mitsamt umgebender Flüssigkeit durch die erste Öffnung in die Vorrichtung eingesaugt wird, indem Flüssigkeit durch die zweite Öffnung abgesaugt bzw. ein Unterdruck angelegt wird. Danach lässt sich das Transplantat durch die zweite, kleinere Öffnung in den Körper des Patienten einbringen, indem Flüssigkeit durch die erste Öffnung zugeführt bzw. ein geeigneter Überdruck aufgebaut wird.

Zwischen der ersten, größeren Öffnung und der zweiten, kleineren Öffnung der Vorrichtung nimmt der Querschnitt der Vorrichtung ab. In erfindungsgemäßer Weise lässt sich das Transplantat so besonders schonend von der ersten Öffnung zur zweiten Öffnung verlagern, um in den Patienten eingebracht zu werden. Der Übergang zwischen der ersten und der zweiten Öffnung lässt sich dabei so ausgestalten, dass bspw. ein automatisches Zusammenrollen des Transplantats stattfindet, nämlich insbesondere in Bezug auf die Verpflanzung von Hornhautschichten. So ist eine grundlegende Erleichterung für den durchführenden Operateur geschaffen.

Mit der Vorrichtung ist eine Standardisierung bestimmter Eingriffe, bspw. von DMEK-Operationen ermöglicht. Eine ggf. sehr schwierige Präparation von Spendergewebe, insbesondere von Hornhautschichten, kann durch Transplantations- bzw. Hornhautbanken vorgenommen und das Spendergewebe bereits in geeigneter Weise in einer im Einwegverfahren zu benutzenden erfindungsgemäßen Vorrichtung vorgelegt werden. Der Operateur kann dann nach Bedarf ein vorsterilisiertes Transplantat bzw. Implantat innerhalb einer erfindungsgemäßen Vorrichtung aus einer sterilen Verpackung entnehmen und direkt danach einen Transplantationseingriff vornehmen. Mit der Präparation und der Bereitstellung des Transplantats hätte der Operateur hierbei nichts zu tun.

Dementsprechend erlaubt die Vorrichtung die vorsterilisierte Lieferung von Transplantaten innerhalb der erfindungsgemäßen Vorrichtung als Einmalprodukt. Aufgrund der entfallenden Präparation und des vereinfachten Einbringens des Transplantats während des Eingriffs können so Hemmschwellen und auch finanzielle Vorbehalte der Operateure gegenüber neuartigen Operationsmethoden, insbesondere der DMEK-Operation, abgebaut werden.

Im Ergebnis ist durch die Erfindung eine Vorrichtung zum schonenden Bereitstellen und exakten Einbringen eines Transplantats oder eines Implantats in den lebenden Körper geschaffen worden.

In einer ersten, ganz besonders bevorzugten Ausgestaltung des Erfindungsgedankens ist der äußere Rand der zweiten Öffnung der Vorrichtung in die Düse einer Spritze, insbesondere einer Einwegspritze, und insbesondere einer Einwegspritze mit 5 ml Volumen, einsteckbar, und/oder ist die erste Öffnung der Vorrichtung auf die Düse einer Spritze, insbesondere einer Einwegspritze, und insbesondere einer Einwegspritze mit 5 ml Volumen, aufsteckbar. Mit dieser Weiterbildung ist eine besonders einfach und standardisiert einsetzbare Vorrichtung angegeben. In besonders raffinierter Weise kann der äußere Rand der zweiten Öffnung so ausgestaltet sein, dass dieser in die Düse einer bekannten Einwegspritze einsteckbar ist. So kann das Transplantat (mitsamt Flüssigkeit) durch die erste Öffnung in die Vorrichtung eingezogen werden, indem der Kolben der an der zweiten Öffnung angeschlossenen Spritze nach hinten gezogen wird.

Alternativ, in bevorzugter Weise jedoch zusätzlich, kann die erste, größere Öffnung auf die Düse einer solchen herkömmlichen Spritze aufsteckbar sein. Auf diese Weise kann das innerhalb der Vorrichtung bereitgestellte Transplantat während des Eingriffs in den Körper des Patienten eingebracht werden, indem die erste Öffnung auf die Düse einer Spritze aufgesteckt wird und durch Betätigung des Kolbens der Spritze Flüssigkeit in die Vorrichtung gepresst wird. Dadurch kann im Inneren der Vorrichtung vorhandene Flüssigkeit zusammen mit dem Transplantat durch eine geeignete Inzision in den Körper des Patienten gefördert werden.

In besonders vorteilhafter Weise können Bereitstellen und Einbringen eines Transplantats praktisch unmittelbar hintereinander erfolgen. Zunächst kann das Transplantat über eine auf die zweite Öffnung der Vorrichtung gesteckte Spritze durch die erste Öffnung in die Vorrichtung eingezogen werden. Die Spritze wird daraufhin vom zweiten Ende entfernt und nunmehr in die erste Öffnung eingesteckt. Durch Fördern von Flüssigkeit aus der Spritze in die erste Öffnung kann dann das Transplantat in bestimmungsgemäßer Weise in den Körper des Patienten eingebracht werden.

Es sei darauf hingewiesen, dass der äußere Rand bzw. der Außendurchmesser der zweiten Öffnung auch als schmales Ende der Vorrichtung sowie die erste Öffnung auch als Innendurchmesser des breiten Endes der Vorrichtung bezeichnet werden kann. Die oben verwendeten Begriffe sind insoweit nicht einschränkend zu verstehen.

Insbesondere bezüglich der letztgenannten Ausführungsformen der Erfindung hat sich im Konkreten eine Ausgestaltung als vorteilhaft erwiesen, bei der die erste Öffnung einen Innendurchmesser von ca. 5 mm aufweist und/oder die zweite Öffnung einen Außendurchmesser von ca. 1,75 mm aufweist. So sind die Öffnungen in vorbeschriebener Weise dazu geeignet, an eine handelsübliche Einwegspritze mit 5 ml Volumen angeschlossen zu werden.

Gemäß einer weiteren vorteilhaften Ausgestaltung weist die Vorrichtung einen - insbesondere mittigen - konischen Abschnitt auf und/oder ist die erste Öffnung in einem ggf. zylindrischen ersten Abschnitt der Vorrichtung ausgebildet und/oder ist die zweite Öffnung in einem leicht konischen oder zylindrischen zweiten Abschnitt der Vorrichtung ausgebildet. Hinsichtlich eines schonenden Bereitstellens und Einbringens eines Transplantats bzw. Implantats ist es besonders zweckmäßig, die Querschnittsverringerung der Vorrichtung zwischen der ersten und der zweiten Öffnung zumindest abschnittsweise mittels eines konischen Verlaufs der Innenwand umzusetzen. Das Transplantat kann dadurch ohne Beschädigungen von der ersten zur zweiten Öffnung gefördert werden. Darüber hinaus lässt sich durch eine stetige Querschnittsverringerung ein planmäßiges Zusammenrollen bzw. Zusammenfalten des Transplantats erreichen.

Hinsichtlich der Herstellung einer dichten und spielfreien Verbindung zwischen der ersten Öffnung und der Außenfläche der Düse einer Spritze ist es zweckmäßig, dass die erste Öffnung in einem zylindrischen Abschnitt der Vorrichtung ausgebildet ist. Die zweite Öffnung kann in einem leicht konischen oder zylindrischen Abschnitt der Vorrichtung ausgebildet sein. Über einen zylindrischen Abschnitt lässt sich die zweite Öffnung spielfrei in die Düse einer Spritze einstecken. Allerdings ist der Einführvorgang in die Düse und insbesondere auch das Einbringen eines Transplantats in den Körper des Patienten bei einer leicht konischen Ausbildung dieses Abschnitts erleichtert. Des Weiteren kann sich so eine definierte, umlaufende Dichtlinie zwischen der Außenfläche dieses Abschnitts und der Austrittsöffnung der Düse einer Spritze ergeben.

Insbesondere im Hinblick auf eine Eignung für DMEK-Operationen und/oder für eine bereits beschriebene Ankoppelbarkeit an handelsübliche Einwegspritzen, insbesondere solche mit 5 ml Volumen, kann die Vorrichtung eine Gesamtlänge von ca. 4 mm aufweisen, insbesondere wobei ein der ersten Öffnung zugeordneter Abschnitt eine Länge von ca. 1 mm aufweist und/oder ein mittiger Abschnitt eine Länge von ca. 2 mm aufweist und/oder ein der zweiten Öffnung zugeordneter Abschnitt eine Länge von ca. 1 mm aufweist.

Gemäß einer weiteren vorteilhaften Ausführungsform ist die erfindungsgemäße Vorrichtung aus Kunststoff, insbesondere aus transparentem Kunststoff, oder aus Metall gefertigt. So kann die erfindungsgemäße Vorrichtung insbesondere aus Kunststoffmaterial gefertigt sein, wie er auch für handelsübliche Einwegspritzen verwendet wird. Solche Materialien lassen sich kostengünstig bereitstellen und sind wirksam bereits ab Werk sterilisierbar. Die Wahl eines transparenten Kunststoffs hat dabei den Vorteil, dass die Position des Transplantats/Implantats innerhalb der Vorrichtung beobachtet werden kann. Die Fertigung der erfindungsgemäßen Vorrichtung aus Metall hätte den Vorteil einer größeren Robustheit und der Wiederverwendbarkeit. Dazu könnte eine Vorrichtung aus Metall problemlos durch den Benutzer mittels Erhitzen sterilisiert werden.

In einer weiteren, besonders vorteilhaften Weiterbildung des Erfindungsgedankens weist die Vorrichtung auf der Innenseite eine adhäsionshemmende Substanz, insbesondere Heparin, auf. Dadurch wird ein Anhaften des Transplantats bzw. Implantats an der Innenseite der Vorrichtung wirksam vermieden. So lässt sich die Durchführbarkeit des geplanten Eingriffs sicherstellen und wird insbesondere bei der Verwendung von Spendergewebe eine Beschädigung desselben durch ein Anhaften und darauffolgendes Losreißen von der Innenwand der Vorrichtung vermieden.

Die oben aufgezeigte Aufgabe ist hinsichtlich eines gebrauchsfertigen Sets mit den Merkmalen des Anspruchs 9 gelöst. Danach umfasst das gebrauchsfertige Set eine erfindungsgemäße Vorrichtung nebst Spritze in einer sterilen Verpackung. So lässt sich dem Operateur eine gebrauchsfertige Vorrichtung nebst Spritze zur Durchführung eines geplanten operativen Eingriffs zur Verfügung stellen. In zweckmäßiger Weise kann die Vorrichtung durch eine übliche Sterilisationsmethode behandelt sein, bspw. durch eine Strahlensterilisation. Die erfindungsgemäße Vorrichtung kann - wie eine Einwegspritze oder eine Einwegkanüle - in sterilem Zustand aus der Verpackung entnommen und direkt als Einwegprodukt für den operativen Eingriff benutzt werden.

Innerhalb des erfindungsgemäßen gebrauchsfertigen Sets bietet die erfindungsgemäße Vorrichtung sämtliche Vorteile, welche bereits obenstehend beschrieben worden sind. Auf diese obigen Ausführungen wird daher zur Vermeidung von Wiederholungen verwiesen.

Des Weiteren kann das erfindungsgemäße gebrauchsfertige Set jede der oben ausführlich beschriebenen vorteilhaften Ausgestaltungen und Weiterbildungen der erfindungsgemäßen Vorrichtung enthalten, wodurch erneut die bereits beschriebenen Vorteile erhalten werden.

In einer besonders zweckmäßigen Weiterbildung weist das Set eine besonders geeignete Einwegspritze, insbesondere mit 5 ml Volumen auf, mit der die Vorrichtung bedienbar ist. Dazu kann die zweite Öffnung der Vorrichtung bereits in die Düse der enthaltenen Spritze eingesteckt sein. Mit anderen Worten kann eine einzige sterile Verpackung sowohl eine erfindungsgemäße Vorrichtung als auch eine hierzu passende Einwegspritze enthalten, wobei beide Bestandteile des Sets sterilisiert bereitgestellt werden.

In besonders raffinierter Weise kann das gebrauchsfertige Set eine erfindungsgemäße Vorrichtung enthalten, welche bereits ein Transplantat oder ein Implantat enthält. So kann die Vorrichtung bereits das zu verpflanzende Transplantat als "Preloaded"-Produkt enthalten. Ein solches Produkt kann innerhalb des Sets von spezialisierten Anbietern, bspw. Hornhautbanken oder auch Implantatherstellern, in sterilem und sofort verwendbarem Zustand innerhalb einer erfindungsgemäßen Vorrichtung bereitgestellt werden. Der Operateur selber muss sich demnach weder um die Präparation des Gewebes, noch um die Vorbereitung des Gewebes/Implantats oder eine Sterilisierung kümmern. Eine erfindungsgemäße Vorrichtung als "Preloaded"-Produkt kann innerhalb des gebrauchsfertigen Sets bereits mit der ersten Öffnung an eine Einwegspritze angeschlossen bereitgestellt werden, wobei Vorrichtung und Spritze mit einer geeigneten Flüssigkeit befüllt sind. Die Vorrichtung braucht dann nur noch aus der sterilen Verpackung entnommen zu werden, durch Betätigung der bereits an die Vorrichtung angeschlossenen Spritze lässt sich das Transplantat bzw. das Implantat danach direkt durch eine Inzision in den Körper des Patienten einbringen.

Es gibt nun verschiedene Möglichkeiten, die Lehre der vorliegenden Erfindung in vorteilhafter Weise auszugestalten und weiterzubilden. Dazu ist einerseits auf die nachgeordneten Ansprüche und andererseits auf die nachfolgende Erläuterung eines bevorzugten Ausführungsbeispiels der erfindungsgemäßen Vorrichtung zum Bereitstellen und Einbringen eines Transplantats oder eines Implantats in den lebenden Körper anhand der Zeichnung zu verweisen. In Verbindung mit der Erläuterung des bevorzugten Ausführungsbeispiels der erfindungsgemäßen Vorrichtung anhand der Zeichnung werden auch im Allgemeinen bevorzugte Ausgestaltungen und Weiterbildungen der Lehre erläutert. In der Zeichnung zeigen
- Fig. 1: eine seitliche Schnittdarstellung eines bevorzugten Ausführungsbeispiels der erfindungsgemäßen Vorrichtung, wobei im unteren Teil der Figur das schmale Ende der Vorrichtung mit der zweiten Öffnung noch einmal separat und um 90° gedreht dargestellt ist,
- Fig. 2: in einer schematischen Abfolge dargestellt die Verwendung der erfindungsgemäßen Vorrichtung im Zusammenspiel mit einer handelsüblichen Einwegspritze, um ein Implantat, hier Hornhautschichten für eine DMEK-Operation, innerhalb der Vorrichtung bereitzustellen, und
- Fig. 3: ebenfalls in einer schematisch dargestellten Abfolge die weitere Verwendung der erfindungsgemäßen Vorrichtung mitsamt einer Einwegspritze zum Einbringen des Transplantats in den Körper des Patienten, nämlich hier zur Durchführung einer DMEK-Operation.

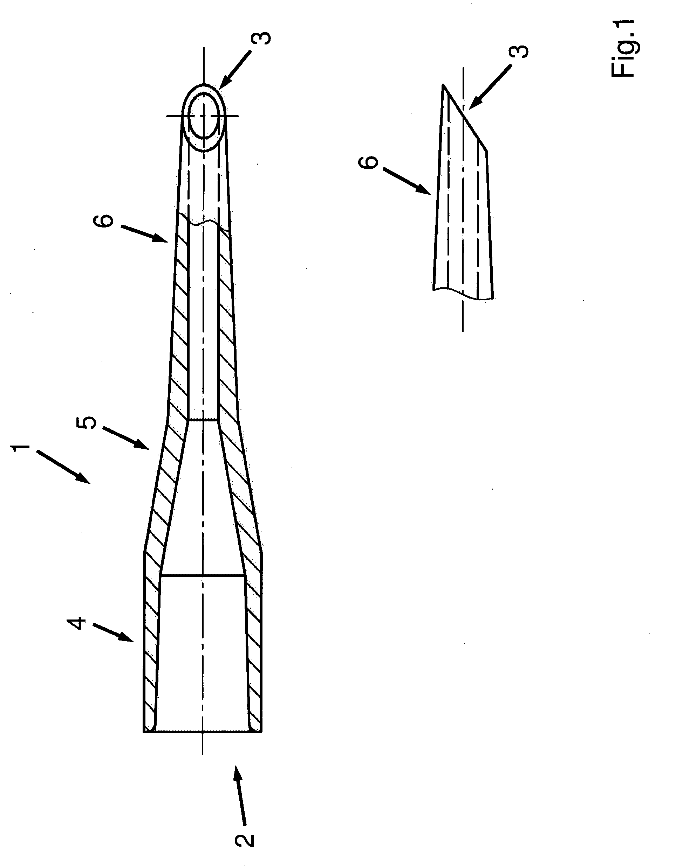

Fig. 1 zeigt oben eine seitliche Schnittdarstellung eines bevorzugten Ausführungsbeispiels der erfindungsgemäßen Vorrichtung, welche hier als Kartusche 1 ausgestaltet ist. Diese Kartusche 1 ist im vorliegenden Beispiel aus transparentem Kunststoff gefertigt, wie er bspw. für die Herstellung herkömmlicher Einwegspritzen verwendet wird.

Die Vorrichtung weist zwei gegenüberliegende, endseitige Öffnungen 2, 3 auf. In erfindungsgemäßer Weise weist die erste Öffnung 2 dabei einen größeren Durchmesser auf als die zweite Öffnung 3. Gemäß der vorliegenden Ausgestaltung lässt sich der Aufbau der erfindungsgemäßen Vorrichtung in Form der Kartusche 1 in drei längs unterteilte Abschnitte 4, 5, 6 gliedern. Ein erster Abschnitt 4 ist der ersten Öffnung 2 zugeordnet. Dieser Abschnitt weist eine zylinderförmige Außenwand auf. Die Innenfläche dieses ersten Abschnitts 4 verjüngt sich über die Länge der Vorrichtung gesehen leicht. So lässt sich der erste Abschnitt 4 dicht und spielfrei auf die Düse einer Spritze aufstecken.

Der mittige Abschnitt 5 der Kartusche 1 verjüngt sich sowohl außen als auch innen stark, um einen Übergang von der ersten Öffnung 2 zur zweiten Öffnung 3 zu schaffen. Aufgrund der konischen Ausgestaltung dieses Abschnitts 5 lässt sich ein Implantat bzw. Transplantat beschädigungsfrei von der ersten Öffnung 2 zur zweiten Öffnung 3 transportieren (insbesondere wobei die Vorrichtung mit einer geeigneten Flüssigkeit gefüllt ist) und auch innerhalb der Vorrichtung bereitstellen.

Der zweite Abschnitt 6, welcher der zweiten Öffnung 3 der Vorrichtung zugeordnet ist, verjüngt sich auf der Außenseite ganz leicht, um im Bereich der zweiten Öffnung 3 das Einführen der Vorrichtung in eine möglichst kleine Inzision im Körper des Patienten zu erleichtern. Auf der Innenseite verläuft dieser Abschnitt 6 beim gezeigten Ausführungsbeispiel zylindrisch.

Im unteren Teil von Fig. 1 ist das schmale Ende der Vorrichtung mit der zweiten Öffnung 3 noch einmal separat und um 90° gedreht in einer Schnittansicht dargestellt. Hierbei ist gut zu erkennen, wie sich die Wandstärke der Vorrichtung im zweiten Abschnitt 6 in Richtung der zweiten Öffnung 3 verjüngt, während die Innenfläche dieses Abschnitts 6 eine zylindrische Form beschreibt. Die von der zweiten Öffnung 3 gebildete Ebene weicht deutlich von einer Senkrechten zur Längsachse der Vorrichtung ab. Die dadurch am vorderen, oberen Ende der zweiten Öffnung 3 entstehende Spitze erleichtert ein Einführen in eine Inzision im Körper des Patienten. Des Weiteren wird so die Durchtrittsfläche für das Transplantat bzw. das Implantat aus der zweiten Öffnung 3 vergrößert, obwohl der Innendurchmesser der zweiten Öffnung 3 senkrecht zur Längsachse der Vorrichtung gesehen sehr gering ist.

Der zweite Abschnitt 6 der Vorrichtung ist so bemessen, dass sich dieser in die Düse einer Einwegspritze einführen lässt, um das Transplantat bzw. das Implantat durch die erste Öffnung 2 durch die Betätigung einer an der zweiten Öffnung 3 angeschlossenen Spritze (hier nicht dargestellt) in den Innenraum der Vorrichtung einzusaugen.

Ebenso lässt sich die erste Öffnung 2 auf die Düse einer Einwegspritze aufstecken.

Ganz speziell ist im vorliegenden Ausführungsbeispiel eine entsprechende Kompatibilität mit herkömmlichen Einwegspritzen mit 5 ml Volumen gegeben. Dazu weist die erste Öffnung 2 einen Innendurchmesser von ca. 5 mm auf, und weist die zweite Öffnung 3 einen Außendurchmesser (senkrecht zur Längsachse gesehen) von ca. 1,75 mm auf.

Zur Schonung eines innerhalb der Vorrichtung bereitgestellten Transplantats ist diese auf der Innenseite mit der adhäsionshemmenden Substanz Heparin beschichtet.

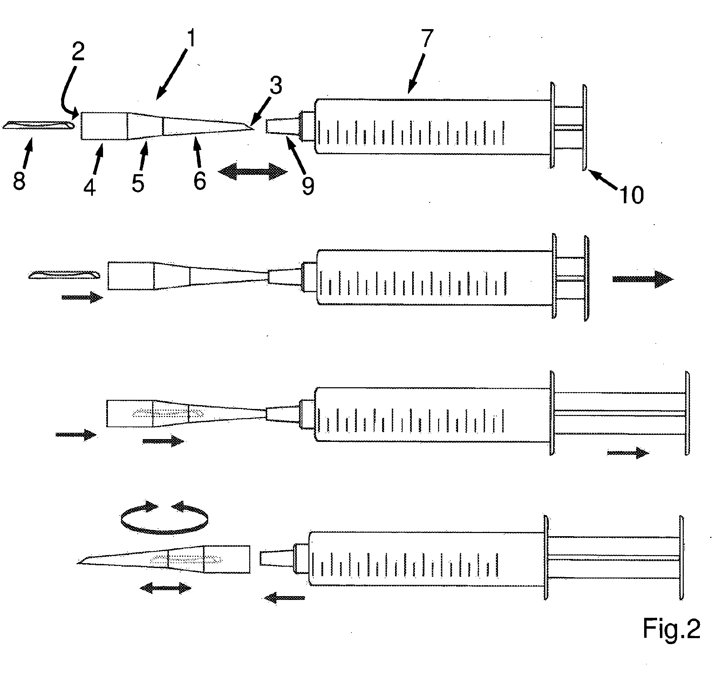

Fig. 2 zeigt in einer schematischen Abfolge (von oben nach unten dargestellt), die Verwendung der erfindungsgemäßen Vorrichtung, nämlich der Kartusche 1, im Zusammenspiel mit einer handelsüblichen Spritze 7, insbesondere einer handelsüblichen Einwegspritze. Dabei wird ein Transplantat 8, nämlich Spendergewebe aus Hornhautschichten für einen DMEK-Eingriff, verwendet. Insbesondere handelt es sich dabei im gezeigten Beispiel um die Descemet-Membran mit dem Endothel. Selbstverständlich kann innerhalb der erfindungsgemäßen Vorrichtung auch jedes andere geeignete Spendergewebe oder Implantat bereitgestellt werden.

Die Kartusche 1 wird mit der zweiten Öffnung 3, d.h. mit dem zweiten Abschnitt 6, in die Düse 9 der Spritze 7 eingesteckt (oberste Darstellung). Dabei wird zwischen dem zweiten Abschnitt 6 und der Düse 9 ein fester Sitz mit ausreichender Dichtigkeit erzielt.

Danach kann über ein Herausziehen des Kolbens 10 der Spritze 7 innerhalb der Kartusche 1 ein Unterdruck erzeugt werden. So wird das Transplantat 8 unter Flüssigkeit oder unter Beigabe von Flüssigkeit in die erfindungsgemäße Vorrichtung eingezogen (zweite Darstellung von oben).

In der dritten Darstellung ist zu sehen, dass das Transplantat 8 aufgrund eines weitergehenden Aufziehens der Spritze 7 inmitten der Kartusche 1 bereitgestellt ist, und zwar im Bereich des mittigen Abschnitts 5.

Die unterste Darstellung dieser schematischen Abfolge zeigt bereits die Vorbereitung des eigentlichen Transplantationseingriffs. Die erfindungsgemäße Vorrichtung, nämlich die Kartusche 1, wird mit dem zweiten Abschnitt 6 aus der Düse 9 der Spritze 7 herausgezogen und umgedreht. Nunmehr kann die erste Öffnung 2 bzw. der erste Abschnitt 4 der Kartusche 1 auf die Düse 9 der Spritze 7 aufgesteckt werden. Es sei nochmals betont, dass dabei sowohl innerhalb der Kartusche 1 als auch im Inneren der Spritze 7 eine geeignete Flüssigkeit vorhanden ist, welche hier nicht dargestellt ist.

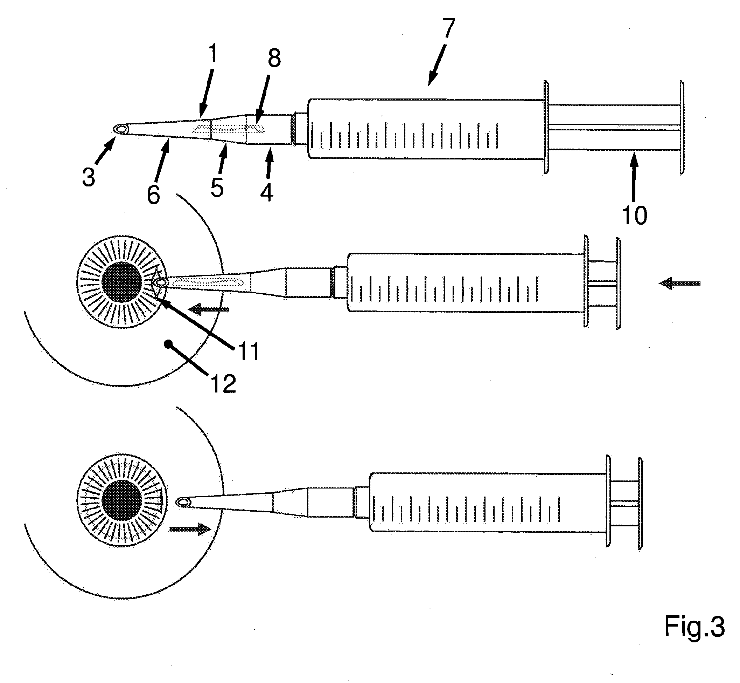

Fig. 3 zeigt ebenfalls in einer schematischen Abfolge (von oben nach unten) die weitere Verwendung der erfindungsgemäßen Vorrichtung mitsamt der Spritze 7 zum Einbringen des Transplantats 8 in den Körper des Patienten, nämlich in eine Inzision 11 im Auge 12 des Patienten zur Durchführung einer DMEK-Operation. In der oberen Darstellung dieser Figur ist die Kartusche 1 mit dem bereitgestellten Transplantat 8 mit der größeren, ersten Öffnung 2 an die Düse 9 der Spritze 7 angeschlossen worden. Damit ist die erfindungsgemäße Vorrichtung bereit zum Einbringen des Transplantats 8 in den Körper des Patienten.

In der mittleren Darstellung ist zu sehen, wie die kleinere, zweite Öffnung 3 der Kartusche 1 in eine vergleichsweise kleine Inzision 11 im Auge 12 des Patienten eingeführt wird. Auf eine perforierende Keratoplastik wird dabei verzichtet. Der Kolben 10 der Spritze 7 wird wieder in den Spritzenkörper zurückgeführt. Durch das Ausfördern von Flüssigkeit aus der Spritze 7 und der Kartusche 1 wird das Transplantat 8 zwangsläufig zuerst in den zweiten Abschnitt 6 der Vorrichtung und schließlich über die zweite Öffnung 3 aus der Vorrichtung heraus durch die Inzision 11 in das Auge 12 des Patienten eingebracht. Durch die zylindrisch bzw. konisch zulaufenden Innenflächen der Kartusche 1 wird dabei eine Beschädigung des Transplantats 8 vermieden. Des Weiteren verhindert eine Innenbeschichtung mit Heparin ein Hängenbleiben des Spendergewebes innerhalb der Vorrichtung.

Nach dem Einbringen des Transplantats 8 in das Auge 12, nämlich in die vordere Augenkammer, kann dieses in geeigneter Weise durch sehr vorsichtige Manipulation ausgebreitet werden. Danach lässt sich das Transplantat 8 mittels Luftzugabe am hinteren Stroma fixieren.

Nach dem erfolgreichen Einbringen des Transplantats 8 wird die Kartusche 1 mitsamt der Spritze 7 aus der Inzision 11 zurückgezogen (unterste Darstellung von Fig. 3). Die Inzision 11 lässt sich danach - sofern nötig - in geeigneter Weise nachversorgen. Die Kartusche 1 sowie die Spritze 7 werden nach dem Eingriff verworfen.

Hinsichtlich weiterer vorteilhafter Ausgestaltungen der erfindungsgemäßen Vorrichtung sowie des erfindungsgemäßen gebrauchsfertigen Sets wird zur Vermeidung von Wiederholungen auf den allgemeinen Teil der Beschreibung sowie auf die beigefügten Ansprüche verwiesen.

Schließlich sei angemerkt, dass das voranstehend erörterten Ausführungsbeispiel der erfindungsgemäßen Vorrichtung zum Bereitstellen und zum Einbringen eines Transplantats oder eines Implantats in den lebenden Körper lediglich zur Erörterung der beanspruchten Lehre dient, dieses - und in diesem Zusammenhang auch das erfindungsgemäße gebrauchsfertige Set - jedoch nicht auf das Ausführungsbeispiel einschränkt.

### Bezugszeichenliste

- 1: Kartusche
- 2: erste Öffnung
- 3: zweite Öffnung
- 4: erster Abschnitt (erste Öffnung)
- 5: mittiger Abschnitt
- 6: zweiter Abschnitt (zweite Öffnung)
- 7: Spritze
- 8: Transplantat
- 9: Düse (Spritze)
- 10: Kolben (Spritze)
- 11: Inzision (Auge)
- 12: Auge

## Patentansprüche

1. Vorrichtung zum Bereitstellen und zum Einbringen eines Transplantats (8) oder eines Implantats in den lebenden Körper, in Kombination mit einer Düse und einem Kolben aufweisenden Spritze zum Betätigen der Vorrichtung, insbesondere für ophthalmologische Eingriffe, und insbesondere zur Durchführung einer DMEK-Operation, wobei die Vorrichtung eine Kartusche (1) mit zwei gegenüberliegenden, endseitigen Öffnungen (2, 3) aufweist, wobei die erste Öffnung (2) einen größeren Durchmesser aufweist als die zweite Öffnung (3), wobei der äußere Rand der zweiten Öffnung (3) in die Düse (9) der Spritze (7) einsteckbar ist und die erste Öffnung (2) auf die Düse (9) der Spritze (7) aufsteckbar ist, so dass das Transplantat (8) oder Implantat mitsamt Flüssigkeit durch die erste Öffnung (2) in die Kartusche (1) eingezogen wird, indem der Kolben der an die zweite Öffnung (3) angeschlossenen Spritze (7) nach hinten gezogen wird, und das Transplantat (8) oder Implantat mitsamt der im Inneren der Vorrichtung vorhandenen Flüssigkeit aus der Kartusche (1) ausstoßbar ist, indem die erste Öffnung (2) auf die Düse (9) der Spritze (7) aufgesteckt und durch Betätigen des Kolbens der Spritze (7) Flüssigkeit in die Kartusche (1) gepresst wird, wodurch die im Inneren der Vorrichtung vorhandene Flüssigkeit zusammen mit dem Transplantat (8) oder Implantat durch eine geeignete Inzision in den Körper eines Patienten befördert wird.

2. Vorrichtung nach Anspruch 1, **dadurch gekennzeichnet, dass** die Spritze (7) als Einwegspritze, insbesondere als Einwegspritze mit 5 ml Volumen, ausgeführt ist.

3. Vorrichtung nach Anspruch 1 oder 2, **dadurch gekennzeichnet, dass** die erste Öffnung (2) einen Innendurchmesser von ca. 5 mm aufweist und/oder dass die zweite Öffnung (3) einen Außendurchmesser von ca. 1,75 mm aufweist.

4. Vorrichtung nach einem der Ansprüche 1 bis 3, **dadurch gekennzeichnet, dass** die Vorrichtung einen - insbesondere mittigen - konischen Abschnitt (5) aufweist, und/oder dass die erste Öffnung (2) in einem zylindrischen ersten Abschnitt (4) der Vorrichtung ausgebildet ist, und/oder dass die zweite Öffnung (3) in einem leicht konischen oder zylindrischen zweiten Abschnitt (6) der Vorrichtung ausgebildet ist.

5. Vorrichtung nach einem der Ansprüche 1 bis 4, **gekennzeichnet durch** eine Gesamtlänge von ca. 4 mm, insbesondere wobei ein der ersten Öffnung (2) zugeordneter erster Abschnitt (4) eine Länge von ca. 1 mm aufweist und/oder ein mittiger Abschnitt (5) eine Länge von ca. 2 mm aufweist und/oder ein der zweiten Öffnung (3) zugeordneter zweiter Abschnitt (6) eine Länge von ca. 1 mm aufweist.

6. Vorrichtung nach einem der Ansprüche 1 bis 5, **dadurch gekennzeichnet, dass** die Vorrichtung aus Kunststoff, insbesondere aus transparentem Kunststoff, oder aus Metall gefertigt ist.

7. Vorrichtung nach einem der Ansprüche 1 bis 6, **dadurch gekennzeichnet, dass** die Vorrichtung auf der Innenseite eine adhäsionshemmende Substanz, insbesondere Heparin, aufweist.

8. Vorrichtung nach einem der Ansprüche 1 bis 7, **dadurch gekennzeichnet, dass** die Vorrichtung nebst Spritze in einer sterilen Verpackung bereitgestellt ist.

9. Gebrauchsfertiges Set umfassend eine Vorrichtung nach einem der Ansprüche 1 bis 8 in einer sterilen Verpackung.

10. Gebrauchsfertiges Set nach Anspruch 9, **dadurch gekennzeichnet, dass** die Vorrichtung ein Transplantat (8) oder ein Implantat enthält.

## Claims

1. Device for preparing and inserting a transplant (8) or an implant into a living body, in combination with a syringe having a nozzle and a plunger for operating the device, in particular for ophthalmological interventions, and in particular for performing a DMEK operation, wherein the device has a cartridge (1) with two openings (2, 3) at opposite ends, wherein the first opening (2) has a larger diameter than the second opening (3), wherein the outside edge of the second opening (3) can be inserted into the nozzle (9) of the syringe (7) and the first opening (2) can be fitted to the nozzle (9) of the syringe (7), so that the transplant (8) or implant is drawn, together with liquid, through the first opening (2) into the cartridge (1) by pulling back the plunger of the syringe (7) connected to the second opening (3), and the transplant (8) or implant, together with the liquid present inside the device, can be ejected from the cartridge (1) by fitting the first opening (2) to the nozzle (9) of the syringe (7) and pressing liquid into the cartridge (1) by operating the plunger of the syringe (7), whereby the liquid present inside the device is delivered together with the transplant (8) or implant through a suitable incision into the body of a patient.

2. Device according to claim 1, **characterised in that** the syringe (7) is in the form of a disposable syringe, in particular in the form of a disposable syringe having a volume of 5 ml.

3. Device according to claim 1 or 2, **characterised in that** the first opening (2) has an inside diameter of approximately 5 mm and/or **in that** the second opening (3) has an outside diameter of approximately 1.75 mm.

4. Device according to any one of claims 1 to 3, **characterised in that** the device has a conical portion (5) - in particular a conical middle portion - and/or **in that** the first opening (2) is formed in a cylindrical first portion (4) of the device, and/or **in that** the second opening (3) is formed in a slightly conical or cylindrical second portion (6) of the device.

5. Device according to any one of claims 1 to 4, **characterised by** an overall length of approximately 4 mm, in particular wherein a first portion (4) associated with the first opening (2) has a length of approximately 1 mm and/or a middle portion (5) has a length of approximately 2 mm and/or a second portion (6) associated with the second opening (3) has a length of approximately 1 mm.

6. Device according to any one of claims 1 to 5, **characterised in that** the device is made of plastics material, in particular of transparent plastics material, or of metal.

7. Device according to any one of claims 1 to 6, **characterised in that** the device has an adhesion-inhibiting substance, in particular heparin, on the inside.

8. Device according to any one of claims 1 to 7, **characterised in that** the device together with the syringe is provided in a sterile packaging.

9. Ready-to-use set comprising a device according to any one of claims 1 to 8 in a sterile packaging.

10. Ready-to-use set according to claim 9, **characterised in that** the device contains a transplant (8) or an implant.

## Revendications

1. Dispositif de préparation et d'insertion d'un greffon (8) ou d'un implant dans le corps vivant, en combinaison avec une pointe comprenant buse et un piston pour l'actionnement du dispositif, plus particulièrement pour des opérations ophtalmologiques et plus particulièrement pour la réalisation d'une opération DMEK, le dispositif comprenant une cartouche (1) avec deux ouvertures terminales (2, 3) opposées, la première ouverture (2) présentant un diamètre supérieur à celui de la deuxième ouverture (3), le bord extérieur de la deuxième ouverture (3) pouvant être inséré dans la buse (9) de la pointe (7) et la première ouverture (2) pouvant être emboîtée sur la buse (9) de la pointe (7), de façon à ce que le greffon (8) ou l'implant soit tiré, en même temps qu'un liquide, à travers la première ouverture (2) dans la cartouche (1), grâce au fait que le piston de la pointe (7) raccordée à la deuxième ouverture (3) est tiré vers l'arrière et le greffon (8) ou l'implant puisse être éjecté hors de la cartouche (1) en même temps que le liquide se trouvant à l'intérieur du dispositif, grâce au fait que la première ouverture (2) est emboîtée sur la buse (9) de la pointe (7) et, grâce à l'actionnement du piston de la pointe (7), un liquide est pressé vers la cartouche (1), ce qui permet de transporter se trouvant à l'intérieur du dispositif vers le corps d'un patient en même temps que le greffon (8) ou l'implant à travers une incision adaptée.

2. Dispositif selon la revendication 1, **caractérisé en ce que** la pointe (7) est réalisée sous la forme d'une seringue à usage unique, plus particulière sous la forme d'une seringue à usage unique avec un volume de 5 ml.

3. Dispositif selon la revendication 1 ou 2, **caractérisé en ce que** la première ouverte (2) présente un diamètre intérieur d'environ 5 mm et/ou **en ce que** la deuxième ouverture (3) présente un diamètre extérieur d'environ 1,75 mm.

4. Dispositif selon l'une des revendications 1 à 3, **caractérisé en ce que** le dispositif présente une portion conique (5), plus particulièrement centrale et/ou **en ce que** la première ouverture (2) est réalisée dans une première portion cylindrique (4) du dispositif et/ou **en ce que** la deuxième ouverture (3) est réalisée dans une deuxième portion (6), légèrement conique ou cylindrique, du dispositif.

5. Dispositif selon l'une des revendications 1 à 4, **caractérisé par** une longueur totale d'environ 4 mm, plus particulièrement une première portion (4) correspondant à la première ouverture (2) présentant une longueur d'environ 1 mm et/ou une portion centrale (5) présentant une longueur d'environ 2 mm et/ou d'une deuxième portion (6) correspondant à la deuxième ouverture (3) présentant une longueur d'environ 1 mm.

6. Dispositif selon l'une des revendications 1 à 5, **caractérisé en ce que** le dispositif est constitué de matière plastique, plus particulièrement d'une matière plastique transparente, ou de métal.

7. Dispositif selon l'une des revendications 1 à 6, **caractérisé en ce que** le dispositif comprend, à l'intérieur, une substance anti-adhésive, plus particulièrement de l'héparine.

8. Dispositif selon l'une des revendications 1 à 7, **caractérisé en ce que** le dispositif est conditionné avec une seringue dans un emballage stérile.

9. Kit prêt à l'emploi comprenant un dispositif selon l'une des revendications 1 à 8 dans un emballage stérile.

10. Kit prêt à l'emploi selon la revendication 9, **caractérisé en ce que** le dispositif contient un greffon (8) ou un implant.
